# EUROPEAN PATENT APPLICATION

(11) **EP 3 835 425 A1**
(43) Date of publication of application: **16.06.2021**
(21) Application number: 20739840.5
(22) Date of filing: 16.06.2020
(51) Int. Cl.: C12N 15/82, C07K 14/005, G01N 33/569, G01N 33/68

(54) **RECOMBINANT VECTOR FOR PRODUCING ANTIGEN FOR DIAGNOSIS OF AFRICAN SWINE FEVER AND USE THEREOF**

(30) Priority: 17.06.2019 KR 20190071861; 15.06.2020 KR 20200072204
(71) Applicant: Bioapplications Inc., Pohang-si, Gyeongsangbuk-do 37668 (KR)
(72) Inventor: SOHN, Eun-Ju, Pohang-Si Gyeongsangbuk-do 37668 (KR); LEE, Sangmin, Pohang-Si Gyeongsangbuk-do 37558 (KR); KANG, Hyang Ju, Pohang-si Gyeongsangbuk-do 37780 (KR)
(74) Representative: Dehns
(86) International application number: PCT/KR2020/007762
(87) International publication number: WO 2020/256372

(57) **Abstract**

The present invention relates to a recombinant vector including a polynucleotide encoding an African swine fever virus P32 protein, a transgenic organism transformed with the recombinant vector, a composition and kit for diagnosing African swine fever comprising an African swine fever virus P32 antigen protein isolated from the transgenic organism, and the like.

<110>     BioApplications Inc.

<120>     Recombinant vectors for the preparation of antigens for diagnosis
          of African swine fever and uses thereof

<130>     MPCT20-059

<150>     KR 10-2019-0071861
<151>     2019-06-17

<150>     KR 10-2020-0072204
<151>     2020-06-15

<160>     8

<170>     KoPatentIn 3.0

<210>     1
<211>     555
<212>     DNA
<213>     Artificial Sequence

<220>
<223>     DNA Sequence of P32 antigen


<400>     1
atgaagatgg aagtcatctt taagacagat cttaggtcat cttcacaagt agtattccat          60

gcaggcagcc tttataattg gttctccgtt gaaatcatta actctggacg tatagtgacc         120

acggcaatta agacacttct gtcaactgtt aagtatgaca ttgttaagag tgctagaatc         180

tacgcaggtc agggttatac agaacatcaa gcccaagagg aatggaatat gattctccat         240

gtgctctttg aggaggagac tgagtctgct agttctgaga acattcacga gaagaacgat         300

aatgagacaa atgagtgtac ctcgagtttt gagactttgt tcgaacagga gccttctagc         360

gaggtgccaa aggactccaa gttgtacatg ctggcccaga agaccgttca gcacatcgag         420

cagtacggga aggctcctga ttttaataag gtgattaggg ctcacaactt catacaaact         480

atttatggaa ctcctcttaa ggaggaagag aaggaggttg ttagattgat ggtcataaag         540

ctattaaaga agaag                                                          555


<210>     2
<211>     185
<212>     PRT
<213>     Artificial Sequence

<220>
<223>     Amino acid Sequence of P32 antigen


<400>     2
Met Lys Met Glu Val Ile Phe Lys Thr Asp Leu Arg Ser Ser Ser Gln
  1               5                  10                  15

Val Val Phe His Ala Gly Ser Leu Tyr Asn Trp Phe Ser Val Glu Ile
             20                  25                  30

Ile Asn Ser Gly Arg Ile Val Thr Thr Ala Ile Lys Thr Leu Leu Ser
        35                  40                  45

Thr Val Lys Tyr Asp Ile Val Lys Ser Ala Arg Ile Tyr Ala Gly Gln
    50                  55                  60

Gly Tyr Thr Glu His Gln Ala Gln Glu Glu Trp Asn Met Ile Leu His
65                  70                  75                  80

Val Leu Phe Glu Glu Glu Thr Glu Ser Ala Ser Ser Glu Asn Ile His
                85                  90                  95

Glu Lys Asn Asp Asn Glu Thr Asn Glu Cys Thr Ser Ser Phe Glu Thr
            100                 105                 110

Leu Phe Glu Gln Glu Pro Ser Ser Glu Val Pro Lys Asp Ser Lys Leu
        115                 120                 125

Tyr Met Leu Ala Gln Lys Thr Val Gln His Ile Glu Gln Tyr Gly Lys
    130                 135                 140

Ala Pro Asp Phe Asn Lys Val Ile Arg Ala His Asn Phe Ile Gln Thr
145                 150                 155                 160

Ile Tyr Gly Thr Pro Leu Lys Glu Glu Glu Lys Glu Val Val Arg Leu
                165                 170                 175

Met Val Ile Lys Leu Leu Lys Lys Lys
            180                 185

<210>    3
<211>    251
<212>    DNA
<213>    Artificial Sequence

<220>
<223>    DNA Sequence of BiP

<400>    3
atggctcgct cgtttggagc taacagtacc gttgtgttgg cgatcatctt cttcggtgag        60

tgattttccg atcttcttct ccgatttaga tctcctctac attgttgctt aatctcagaa       120

ccttttttcg ttgttcctgg atctgaatgt gtttgtttgc aatttcacga tcttaaaagg       180

ttagatctcg attggtattg acgattggaa tctttacgat ttcaggatgt ttatttgcgt       240

tgtcctctgc a                                                            251

<210>    4
<211>    18
<212>    DNA
<213>    Artificial Sequence

<220>
<223>    DNA Sequence of 6X His

<400>    4

caccaccatc accaccac                                                    18

<210>    5
<211>    6
<212>    PRT
<213>    Artificial Sequence

<220>
<223>    Amino acid Sequence of 6X His

<400>    5
His His His His His His
  1               5

<210>    6
<211>    12
<212>    DNA
<213>    Artificial Sequence

<220>
<223>    DNA sequence of HDEL

<400>    6
catgatgagc tc                                                          12

<210>    7
<211>    4
<212>    PRT
<213>    Artificial Sequence

<220>
<223>    Amino acid sequence of HDEL

<400>    7
His Asp Glu Leu
  1

<210>    8
<211>    884
<212>    DNA
<213>    Artificial Sequence

<220>
<223>    DNA sequence of full P32 construct

<400>    8
tctagaatta ttacatcaaa acaaaaaatg gctcgctcgt ttggagctaa cagtaccgtt      60

gtgttggcga tcatcttctt cggtgagtga ttttccgatc ttcttctccg atttagatct     120

cctctacatt gttgcttaat ctcagaacct tttttcgttg ttcctggatc tgaatgtgtt     180

tgtttgcaat ttcacgatct taaaaggtta gatctcgatt ggtattgacg attggaatct     240

ttacgatttc aggatgttta tttgcgttgt cctctgcagg atccatgaag atggaagtca     300

tctttaagac agatcttagg tcatcttcac aagtagtatt ccatgcaggc agcctttata 360

attggttctc cgttgaaatc attaactctg gacgtatagt gaccacggca attaagacac 420

ttctgtcaac tgttaagtat gacattgtta agagtgctag aatctacgca ggtcagggtt 480

atacagaaca tcaagcccaa gaggaatgga atatgattct ccatgtgctc tttgaggagg 540

agactgagtc tgctagttct gagaacattc acgagaagaa cgataatgag acaaatgagt 600

gtacctcgag ttttgagact ttgttcgaac aggagccttc tagcgaggtg ccaaaggact 660

ccaagttgta catgctggcc cagaagaccg ttcagcacat cgagcagtac gggaaggctc 720

ctgattttaa taaggtgatt agggctcaca acttcataca aactatttat ggaactcctc 780

ttaaggagga agagaaggag gttgttagat tgatggtcat aaagctatta aagaagaagt 840

cccggggtca ccaccatcac caccatgatg agctctagct cgag 884

## Description

### TECHNICAL FIELD

The present invention relates to a recombinant vector for producing the P32 protein as an antigen for diagnosing African swine fever (ASF), a transgenic organism transformed with the recombinant vector, a composition and kit for diagnosing African swine fever including the P32 protein of an ASF virus isolated from the transgenic organism, and the like.

This application claims priority to and the benefit of Korean Patent Application Nos. 10-2019-0071861, filed on June 17, 2019, and 10-2020-0072204, filed on June 15, 2020, the disclosures of which are incorporated herein by reference in their entirety.

### BACKGROUND ART

African swine fever (ASF) is a swine infectious disease caused by infections with the African swine fever virus (ASFV) belonging to the family *Asfarviridae,* which was first reported in Kenya in 1921 after which there were reports of an outbreak in the sub-Saharan region, and since 2007, the outbreak has begun to expand to regions other than Africa, such as Georgia, Armenia, and Azerbaijan, which are on the Black Sea coast. In particular, in recent years, the outbreak has spread from east to west in Russia, where there is much trade with Korea. It is very important to diagnose African swine fever since it is a high-risk infectious disease that exhibits a mortality rate of 100% when swine infection occurs, and requires immediate disposal after an outbreak due to no vaccine developed for this disease.

Among proteins specific to the African swine fever virus, P15, P35, P72, P54, P30, and the like are known to have high antigenicity, and thus are being considered as candidates for diagnosis. To date, diagnostic kits using antibodies against the African swine fever virus have never been produced, and in foreign countries, antibody diagnostic kits using P30 recombinant proteins (manufactured by SVANOVIR) have been commercialized and are being used.

Meanwhile, due to remarkable advances in molecular biology and genetic engineering techniques, these techniques have also been applied to the field of plants, and thus efforts to produce useful bioactive substances from plants have continued. Producing useful substances from plants can significantly reduce production costs, and is advantageous in that not only various contaminants (viruses, oncogenes, and enterotoxins) that can occur in conventional popular methods (synthesis of proteins from animal cells or microorganisms and isolation and purification thereof) can be fundamentally excluded, but it is also possible to manage seed stock using seeds in a commercialization stage, unlike animal cells or microorganisms.

Therefore, as having made intensive efforts to develop a rapid antigen test for diagnosis of African swine fever in order to maintain a country free of African swine fever and prepare for the influx of disease, the inventors of the present invention developed a system capable of expressing, in a plant, a P32 recombinant protein, among from proteins specific to the African swine fever virus, with high efficiency, and thus completed the present invention.

### DESCRIPTION OF EMBODIMENTS

### TECHNICAL PROBLEM

The African swine fever virus is a giant double-stranded DNA virus that replicates the cytoplasm of infected cells. The virus causes hemorrhagic fever with high mortality rates in pigs, and also persistently infects its natural hosts, pigs, warthogs, bushpigs, and soft ticks of the genus *Ornithodoros,* which likely act as a carrier, with no signs of disease. Since the virus causes fetal hemorrhagic fever in pigs, it is very important to diagnose this virus early.

The present invention has been derived to meet the need for rapid diagnosis of individuals suspected of infection by African swine fever and address the problems of the related art, and an object of the present invention is to provide a recombinant African swine fever virus antigen that not only can be efficiently produced using a plant, but also has high diagnostic sensitivity and specificity, and a composition or kit for diagnosing African swine fever including the same.

Another object of the present invention is to provide a recombinant vector including a gene encoding the African swine fever virus antigen, a transgenic organism transformed with the recombinant vector, and the like.

Another object of the present invention is to provide a method of diagnosing African swine fever, a method of producing a recombinant antigen for the diagnosis of African swine fever, a method of preparing a composition for diagnosing African swine fever including the recombinant protein, and the like.

However, technical problems to be solved by the present invention are not limited to the above-described technical problems, and other unmentioned technical problems will become apparent from the following description to those of ordinary skill in the art.

### TECHNICAL SOLUTION

According to an aspect of the present invention, there is provided a recombinant vector for producing an antigen for the diagnosis of African swine fever, the recombinant vector including a polynucleotide encoding an African swine fever virus P32 protein consisting of an amino acid sequence of SEQ ID NO: 2, and being expressed in a plant.

In one embodiment of the present invention, the recombinant vector may further include a polynucleotide encoding a chaperone binding protein (BiP) of SEQ ID NO: 3.

In another embodiment of the present invention, the polynucleotide encoding a BiP may be located upstream of a 5' terminal of the polynucleotide encoding the P32 protein, but the present invention is not limited thereto.

In another embodiment of the present invention, the recombinant vector may further include a polynucleotide encoding polyhistidine of SEQ ID NO: 4.

In another embodiment of the present invention, the polynucleotide encoding polyhistidine may be located downstream of a 3' terminal of the polynucleotide encoding the P32 protein, but the present invention is not limited thereto.

In another embodiment of the present invention, the recombinant vector may further include a polynucleotide encoding HDEL of SEQ ID NO: 6.

In another embodiment of the present invention, the polynucleotide encoding HDEL may be located downstream of a 3' terminal of the polynucleotide encoding the P32 protein, but the present invention is not limited thereto.

In another embodiment of the present invention, the recombinant vector may further include: a polynucleotide encoding a BiP of SEQ ID NO: 3; a polynucleotide encoding polyhistidine of SEQ ID NO: 4; and a polynucleotide encoding HDEL of SEQ ID NO: 6, but the present invention is not limited thereto.

In another embodiment of the present invention, the recombinant vector may have a polynucleotide encoding a BiP, a polynucleotide encoding the P32 protein, a polynucleotide encoding polyhistidine, and a polynucleotide encoding HDEL, which are sequentially linked to one another, but the present invention is not limited thereto.

In another embodiment of the present invention, the recombinant vector may include a nucleotide sequence of SEQ ID NO: 8, but the present invention is not limited thereto.

The present invention also provides a transgenic organism transformed with the recombinant vector.

In one embodiment of the invention, the transgenic organism may be a plant.

The present invention also provides a recombinant African swine fever virus P32 protein produced using the recombinant vector.

The present invention also provides a use of a recombinant African swine fever virus P32 protein produced using the recombinant vector for diagnosing African swine fever.

The present invention also provides a recombinant African swine fever virus P32 protein produced using the recombinant vector as a use for producing an agent used in the diagnosis of African swine fever.

In one embodiment of the invention, the protein may be water-soluble.

The present invention also provides a composition for diagnosing African swine fever, including, as an active ingredient, the recombinant African swine fever virus P32 protein.

The present invention also provides a kit for diagnosing African swine fever, including, as an active ingredient, the recombinant African swine fever virus P32 protein.

The present invention also provides a method of diagnosing African swine fever or a method of determining African swine fever, the method including detecting an antibody against an African swine fever virus through an antigen-antibody reaction in a biological sample derived from an animal other than a human by using, as an antigen, a recombinant African swine fever virus P32 protein produced using the recombinant vector.

The present invention also provides a method of producing a recombinant antigen for the diagnosis of African swine fever, including: (a) transforming a plant with the recombinant vector according to the present invention; and (b) isolating and purifying a recombinant antigen from the plant.

The present invention also provides a method of preparing a composition or manufacturing a kit for diagnosing African swine fever, including: (a) transforming a plant with the recombinant vector according to the present invention; (b) isolating and purifying a recombinant antigen from the plant; and (c) preparing a diagnostic composition or manufacturing a diagnostic kit using the isolated and purified recombinant antigen.

### ADVANTAGEOUS EFFECTS OF INVENTION

Proteins, especially antigens, for use in the diagnosis and prevention of viral diseases, including African swine fever, cannot use bacteria due to problems such as protein folding and glycosylation, and are produced mainly using animal cells. However, a method of producing a vaccine using animal cells incurs large costs for expanding equipment for mass production, and thus manufacturing is not easy, and in most cases, antigens are expensive. In addition, antigens produced using animal cells are disadvantageous in that storage is not easy, and they are highly likely to be contaminated with viruses that can infect animals. However, the present invention compensates for these disadvantages by using plants. In other words, unlike animal cells, plant cells are highly unlikely to be contaminated with viruses that can infect animals, and mass production is possible at any time by only securing a cultivated area, and long-term storage is possible through plants, and thus it is possible to produce stable and inexpensive antigens.

A recombinant African swine fever virus antigen of the present invention is not only effectively expressed in plants, but also has high water solubility, thus facilitating isolation and purification thereof, and also exhibits high sensitivity and specificity to the African swine fever virus, and thus is expected to be widely used in various fields.

In addition, a composition or kit for diagnosing African swine fever can be produced by using a recombinant African swine fever virus antigen protein according to the present invention, and thus can diagnose individuals infected by African swine fever early and accordingly, can be effectively used to prevent the spread of diseases.

### BRIEF DESCRIPTION OF DRAWINGS

FIG. 1 is a cleavage map showing a gene arrangement for the expression of an African swine fever virus P32 antigen in a plant, according to an embodiment of the present invention (NB, new chaperone binding protein; 6xHis, polyhistidine tag; HDEL, ER retention signal).
FIG. 2 illustrates western blotting results of a P32 antigen after being expressed in a plant, and then isolated and purified (T, Total extract; S, Supernatant fraction; P, Pellet fraction).
FIG. 3 illustrates the results of confirming the purity of an African swine fever virus P32 antigen after being expressed in a plant, according to an embodiment of the present invention.
FIG. 4 illustrates the results of testing the reactivity and sensitivity of an antibody serum diagnostic kit manufactured for African swine fever including a composition of the present invention by using serum provided by a Spanish reference laboratory for African swine fever.

### MODE OF INVENTION

Unless defined otherwise, all technical and scientific terms used herein have the same meanings as commonly understood by one of ordinary skill in the art to which the present invention pertains. In general, the nomenclature used herein is well known and commonly used in the art.

The present invention provides a recombinant vector for producing an antigen for the diagnosis of African swine fever, the recombinant vector including a polynucleotide encoding an African swine fever virus P32 protein, and being expressed in a plant.

The African swine fever virus P32 protein is known to be involved in the internalization of the virus in the early stage of an infection cycle.

In addition, the P32 protein may be encoded by the nucleotide sequence of SEQ ID NO: 1, or may consist of the amino acid sequence of SEQ ID NO: 2, but the present invention is not limited thereto. More specifically, the nucleotide sequence encoding the African swine fever virus P32 protein of the present invention may consist of the nucleotide sequence represented by SEQ ID NO: 1, but present invention is not limited thereto, and variants of the nucleotide sequence fall within the scope of the present invention. A nucleic acid molecule of the nucleotide sequence represented by SEQ ID NO: 1 of the present invention is a functional equivalent of the nucleic acid molecule constituting this, for example, a concept including variants in which some nucleotide sequences of the nucleic acid molecule are modified by deletion, substitution, or insertion, but which can perform functionally the same action as that of the nucleic acid molecule. In particular, the gene may include a nucleotide sequence with at least 70% homology, more preferably at least 80% homology, further more preferably at least 90% homology, and most preferably, at least 95% homology to the nucleotide sequence represented by SEQ ID NO: 1. For example, the gene includes a polynucleotide with sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and 100%. The " sequence homology of %" may be confirmed by comparing two optimized sequences using a comparison region, and some of the polynucleotide sequences in the comparison region may include an addition or deletion (i.e., a gap) compared to reference sequences (additions or deletions excluded) for the optimal alignment of two sequences.

The term "polynucleotide" as used herein refers to an oligomer or polymer including two or more nucleotides or nucleotide derivatives, usually linked by phosphate ester linkages, including deoxyribonucleic acid (DNA) and ribonucleic acid (RNA). Polynucleotides also include, for example, nucleotide analogues, or DNA and RNA derivatives including "skeletal" bonds other than phosphodiester linkages, such as phosphotriester linkages, phosphoramidate linkages, phosphorothioate linkages, thioester linkages, or peptide linkages (peptide nucleic acids). Polynucleotides include single-stranded and/or double-stranded polynucleotides, such as deoxyribonucleic acid (DNA) and ribonucleic acid (RNA), as well as analogues of either RNA or DNA.

In one embodiment of the present invention, the recombinant vector may further include a polynucleotide encoding a chaperone binding protein (BiP), a polynucleotide encoding six consecutive histidines (polyhistidine), a polynucleotide encoding a His-Asp-Glu-Leu (HDEL) peptide, or the like.

In another embodiment of the present invention, the polynucleotide encoding a BiP may be located upstream of a 5' terminal of the polynucleotide encoding the P32 protein, and the polynucleotide encoding polyhistidine and the polynucleotide encoding a HDEL peptide may be located downstream of a 3' terminal of the polynucleotide encoding the P32 protein.

As used herein, the term "recombinant vector" refers to a vector capable of expressing a peptide or protein encoded by a heterologous nucleic acid inserted into the vector, and preferably means a vector constructed so as to express a target antigen (in the present invention, African swine fever antigen P32). The term "vector" as used herein refers to any vehicle for the introduction and/or transfer of a nucleotide into a host cell *in vitro, ex vivo,* or *in vivo,* and may mean a replicon to which another DNA fragment may be attached so as to bring about the replication of the attached fragment. The term "replicon" refers to any genetic unit (e.g., a plasmid, a phage, a cosmid, a chromosome, and a virus) that functions as an autonomous unit of DNA replication *in vivo,* i.e., is capable of replicating by self-regulation.

The recombinant vector of the present invention may include, preferably, a promoter which is a transcription initiation factor to which an RNA polymerase binds, an arbitrary operator sequence for regulating transcription, a sequence encoding a suitable mRNA ribosome binding site, a sequence regulating the termination of transcription and translation, a terminator, or the like, may further include, more preferably, the BiP gene, His-tag (amino acid moiety consisting of at least six histidine residues), an endoplasmic reticulum signal peptide (the same meaning as endoplasmic reticulum-targeting sequence) gene, a cloning site, or the like, and may further include, more preferably, marker genes for selection such as an antibiotic-resistant gene for selecting a transgenic organism.

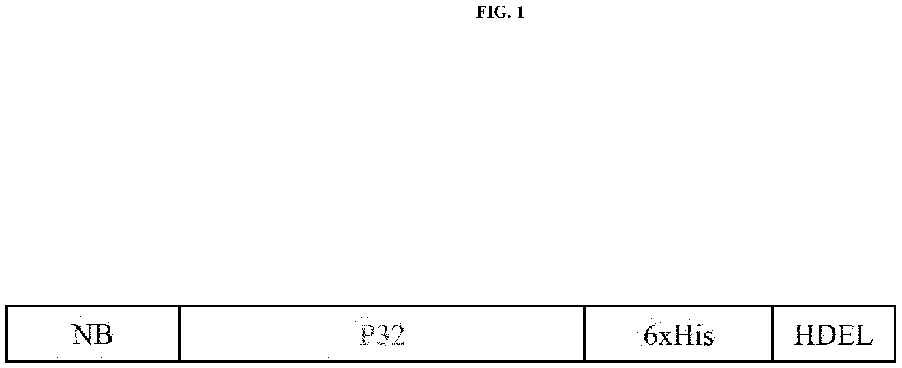

The "gene (illustrated as NB in FIG. 1)" is preferably a gene including a nucleotide sequence of SEQ ID NO: 3, most preferably, a gene represented by SEQ ID NO: 3, but may include a nucleotide sequence with at least 80% homology, more preferably at least 90% homology, and even more preferably at least 95% homology, to the nucleotide sequence of SEQ ID NO: 3. The BiP gene may have only some amino acids remaining by cleavage of a portion of the sequence when a recombinant protein is expressed.

The "cloning site" collectively refers to those inserted for linking/dividing respective genes in a vector.

The type and amino acid sequence of the "endoplasmic reticulum signal peptide (ER signal sequence)" is not limited as long as it is a plant endoplasmic reticulum signal peptide known to those of ordinary skill in the art, and for example, for this, refer to documents such as US 2013/0295065 and WO 2009/158716. In the present invention, the "endoplasmic reticulum signal peptide" may be, preferably, His-Asp-Glu-Leu (HDEL, a polypeptide represented by SEQ ID NO: 7), and may be encoded by a nucleotide sequence represented by SEQ ID NO: 6. In addition, as the endoplasmic reticulum signal peptide of the present invention, a variant of SEQ ID NO: 6 falls within the scope of the present invention. In particular, the gene may include a nucleotide sequence with at least 90% homology, more preferably at least 95% homology, and most preferably at least 98% homology, to the nucleotide sequence of SEQ ID NO: 6. The binding position of the endoplasmic reticulum signal peptide is at the C-terminus of a protein for the purpose of expression or synthesis in plant cells by addition (or linkage).

In the present invention, a suitable gene for a tag, other than the polyhistidine-tag, may include, for example, an Avi tag, a Calmodulin tag, a polyglutamate tag, an E tag, a FLAG tag, a HA tag, a His tag, a Myc tag, an S tag, an SBP tag, an IgG-Fc tag, a CTB tag, a Softag 1 tag, a Softag 3 tag, a Strep tag, a TC tag, a V5 tag, a VSV tag, an Xpress tag, and the like, and the IgG-Fc tag may be derived from a human, a mouse, a rabbit, or a pig.

The marker gene for selection may include, for example, herbicide-resistant genes such as glyphosate and phosphinothricin, antibiotic-resistant genes such as kanamycin, G418, bleomycin, hygromycin, and chloramphenicol, the aadA gene, and the like, the promoter may include, for example, a pEMU promoter, an MAS promoter, a histone promoter, a Clp promoter, a 35S promoter derived from cauliflower mosaic virus, a 19S RNA promoter derived from cauliflower mosaic virus, an actin protein promoter of a plant, an ubiquitin protein promoter, a cytomegalovirus (CMV) promoter, a simian virus 40 (SV40) promoter, a respiratory syncytial virus (RSV) promoter, and an elongation factor-1 alpha (EF-1α) promoter, and the terminator may include, for example, nopaline synthase (NOS) terminator, a rice amylase RAmyl A terminator, a phaseolin terminator, an Octopine gene terminator of *Agrobacterium tumefaciens,* and an *E. coli* rrnB1/B2 terminator, but the present invention is not limited to the above-listed examples.

In another embodiment of the present invention, the recombinant vector may have a promoter gene, a polynucleotide encoding a new chaperone binding protein (BiP; NB) signal peptide, a polynucleotide encoding the P32 protein, a polynucleotide encoding polyhistidine, and a polynucleotide encoding HDEL that are linked in this order.

In the case where the polynucleotides are linked in the above-described order, i.e., in the case of including an expression cassette shown in the cleavage map of FIG. 1, the recombinant vector according to the present invention may include a nucleotide sequence of SEQ ID NO: 8, and consist of, most preferably, the nucleotide sequence of SEQ ID NO: 8, but may include a nucleotide sequence with at least 80% homology, more preferably at least 90% homology, and even more preferably at least 95% homology, to the nucleotide sequence of SEQ ID NO: 8.

Another embodiment of the present invention provides a transgenic organism transformed with the recombinant vector.

In one embodiment of the present invention, the transgenic organism may be a microorganism such as *Escherichia coli, Bacillus, Salmonella,* or yeast, insect cells, animal cells including human cells, an animal such as a mouse, a rat, a dog, a monkey, a pig, a horse, or a cow, *Agrobacterium tumefaciens,* a plant, or the like, and more preferably, examples of the transgenic organism include food crops including rice, wheat, barley, corn, beans, potatoes, red beans, oats, and sorghum; vegetable crops including *Arabidopsis thaliana,* Chinese cabbage, white radish, peppers, strawberries, tomatoes, watermelons, cucumbers, cabbage, oriental melons, pumpkins, spring onions, onions, and carrots; special purpose crops including ginseng, tobacco, cotton, sesame, sugarcane, sugar beets, perilla, peanuts, and rape; fruit crops including apple trees, pear trees, jujube trees, peaches, grapes, tangerines, persimmons, plums, apricots, and bananas; and flowers including roses, carnations, chrysanthemums, lilies, and tulips, but the transgenic organism is not limited as long as it is any living body capable of being transformed with the vector of the present invention.

As used herein, the term "transformation" collectively refers to changes in the genetic properties of an organism by injected DNA, and the term "transgenic organism" refers to a living organism produced by injecting an external gene using a molecular genetic method, and preferably means a living organism transformed by the recombinant vector of the present invention. The living organism is not particularly limited as long as it is a living organism such as microorganisms, eukaryotic cells, insects, animals, plants, and the like, and examples thereof include, but are not limited to, *E. coli, Salmonella, Bacillus,* yeast, animal cells, mice, rats, dogs, monkeys, pigs, horses, cows, *Agrobacterium tumefaciens,* and plants.

As used herein, "plant" may be any plant capable of mass-producing a protein including the antigen of the present invention, and more particularly, may be selected from the group consisting of tobacco, *Arabidopsis thaliana,* corn, soybeans, canola, alfalfa, sunflower, sorghum, wheat, cotton, peanuts, tomatoes, potatoes, lettuce, and pepper, preferably tobacco. In the present invention, tobacco is a plant belonging to the genus *Nicotiana,* the type of tobacco is not particularly limited as long as it is capable of over-expressing a protein, and the present invention may be carried out by selecting an appropriate species in accordance with a transformation method and the purpose of mass production of proteins. For example, a species such as *Nicotiana benthamiana* L. or *Nicotiana tabacum* cv. *Xanthi* may be used.

The transgenic organism may be produced using a method such as transformation, transfection, an *Agrobacterium-mediated* transformation method, particle gun bombardment, sonication, electroporation, and a polyethylene glycol (PEG)-mediated transformation method, but the method is not limited as long as it is a method capable of injecting the vector of the present invention.

Another embodiment of the present invention provides a recombinant African swine fever virus P32 protein (antigen) produced using the recombinant vector according to the present invention.

In one embodiment of the invention, the recombinant P32 protein (antigen) may be water-soluble. More specifically, the recombinant P32 protein expressed in a plant may be 95%, 96%, 97%, 98%, 99%, or 100% dissolved in a water-soluble fraction.

In addition, in another embodiment of the present invention, the recombinant P32 protein (antigen) may be isolated and purified with a purity of 90% or more. More specifically, the recombinant P32 protein expressed in a plant may be obtained as a recombinant P32 protein with a purity of 90%, 91%, 92%, 93%, 94%, 95%, 96%, 97%, 98%, 99%, or 100% when a conventional isolation and purification method is used.

Another embodiment of the present invention provides a composition or kit for diagnosing African swine fever, including, as an active ingredient, the recombinant African swine fever virus P32 protein (antigen) produced using the recombinant vector according to the present invention.

The term "diagnosis" as used herein means determination of the existence of pathological conditions or the possibility of onset thereof. Among these, the present invention is effective in diagnosing, particularly, African swine fever. When a P32 antigen produced by the recombinant vector of the present invention is used, a specific antibody produced in the body of an individual infected by African swine fever may be detected. That is, the P32 antigen may be used as an indicator (diagnostic marker) useful for diagnosing African swine fever.

As used herein, the term "antigen" collectively refers to any substance inducing immune responses *in vivo,* and the antigen is preferably a virus, a compound, a bacterium, pollen, a cancer cell, or a peptide or protein of a part thereof, but is not particularly limited as long as it is a substance capable of inducing an immune response *in vivo.*

Another embodiment of the present invention provides a method of diagnosing African swine fever, including detecting an antibody against an African swine fever virus through an antigen-antibody reaction in a biological sample derived from an animal other than a human by using, as an antigen, a recombinant African swine fever virus P32 protein produced using the recombinant vector according to the present invention.

Another embodiment of the present invention provides a method of producing a recombinant antigen for the diagnosis of African swine fever, including: (a) transforming a plant with the recombinant vector according to the present invention; and (b) isolating and purifying a recombinant antigen from the plant.

Another embodiment of the present invention provides a method of preparing a composition or manufacturing a kit for diagnosing African swine fever, including: (a) transforming a plant with the recombinant vector according to the present invention; (b) isolating and purifying a recombinant antigen from the plant; and (c) preparing a diagnostic composition or manufacturing a diagnostic kit using the isolated and purified recombinant antigen.

Hereinafter, exemplary examples will be described to aid in understanding of the present invention. However, the following examples are provided merely to facilitate the understanding of the present invention and are not intended to limit the scope of the present invention.

### [Examples]

### Example 1: Construction of Recombinant Vector Expressing P32 Antigen of African Swine Fever Virus

As shown in the cleavage map of FIG. 1, a recombinant plant expression vector was constructed so as to express, in a plant, the African swine fever virus antigen P32 protein (known to be involved in the internalization of the virus in the early stage of the infection cycle). More specifically, genetic information for the P32 protein of the African swine fever virus was obtained, and a gene encoding the P32 protein (SEQ ID NO: 1) was synthesized with a sequence optimized for expression in a plant.

Specifically, a polynucleotide (SEQ ID NO: 3) encoding a chaperone binding protein (BiP) signal peptide between a CaMV 35S promoter gene and an NOS terminator of a pCAMBIA1300 vector, a polynucleotide (SEQ ID NO: 1) encoding a recombinant P32 antigen protein of the African swine fever virus, a polynucleotide (SEQ ID NO: 4) encoding His-tag consisting of six consecutive histidines, and a polynucleotide (SEQ ID NO: 6) encoding a His-Asp-Glu-Leu (HDEL) peptide were sequentially linked, thereby completing the construction of a plant expression vector of the P32 protein of the African swine fever virus.

### Example 2: Expression and Identification of P32 Antigen

### 2.1. Transient Expression of African Swine Fever Virus P32 Antigen Vector Expressed in Plant

The plant expression vector prepared in Example 1 was transformed into an *Agrobacterium* LBA4404 strain using electroporation. The transformed *Agrobacteria* was shake-cultured in 5 mL of a YEP liquid medium (yeast extract 10 g, peptone 10 g, NaCl 5 g, kanamycin 50 mg/L, and rifampicin 25 mg/L) at 28 °C for 16 hours, and then 1 mL of the primary culture broth was inoculated into 50 mL of a fresh YEP medium and shake-cultured at 28 °C for 6 hours. The cultured *Agrobacteria* was collected by centrifugation (7,000 rpm, 4 °C, 5 minutes), and then suspended in an infiltration buffer [10 mM MES (pH 5.7), 10 mM MgCl₂, 200 µM acetosyringone] so that absorbance at a wavelength of 600 nm became 1.0. Agro-infiltration was performed by injecting the *Agrobacteria* suspension into the back side of leaves of *Nicotiana benthamiana* using a needleless syringe.

### 2.2. Confirmation of Expression of African Swine Fever Virus P32 Antigen

Proteins were extracted from the plant leaves prepared in Example 2.1 and centrifuged, and then proteins in a water-soluble supernatant (S), proteins in a pellet (P) fraction, and a total (T) fraction including both the water-soluble supernatant and the pellet were separated, followed by western blotting to confirm the expression of a recombinant P32 antigen protein. More specifically, 30 µL of each fraction was mixed with a SDS sample buffer, and then heated. Then, each fraction was subjected to electrophoresis on a 10% SDS-PAGE gel to identify protein bands separated according to size, the separated proteins were transferred to a PVDF membrane, followed by blocking using 5% skim milk, and then the proteins were subjected to binding to antibodies that react with polyhistidine, and treated with an ECL solution using a method provided by the manufacturer to identify the expression of the recombinant P32 antigen protein.

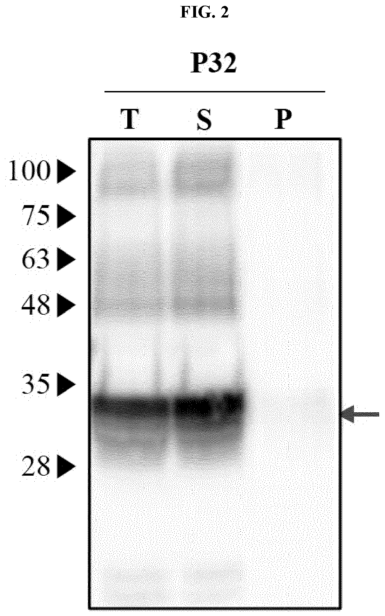

As a result, as illustrated in FIG. 2, it was confirmed that the recombinant P32 antigen protein was expressed with high efficiency, and 95% or more of the expressed recombinant P32 antigen protein was confirmed in the water-soluble supernatant.

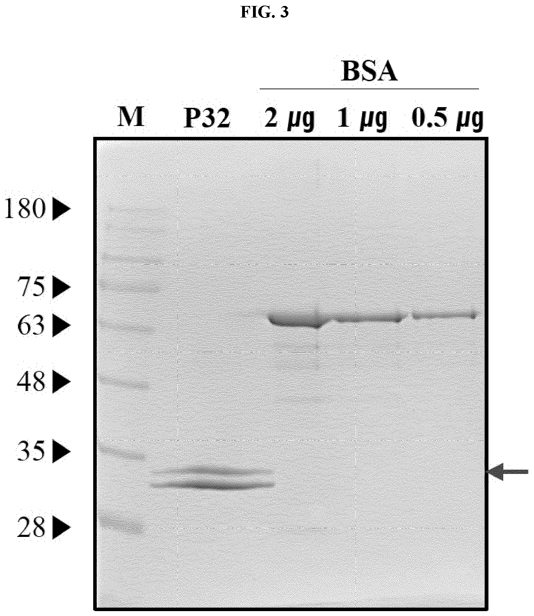

In addition, the SDS-PAGE results of FIG. 3 showed that no additional protein bands other than P32 appeared as can be confirmed in the lane labeled P32, and as a result of quantifying the recombinant protein using a standard curve using bovine serum albumin (BSA), it was confirmed that the P32 antigen protein was isolated with high purity.

As described above, the recombinant African swine fever virus P32 protein of the present invention was well purified without any significant changes or modifications compared to the original protein. These results confirm that, when the protein was expressed in a plant, problems such as mutation of the sugar structure and reduction in production efficiency were not found, and the recombinant African swine fever virus P32 protein according to the present invention is well produced in plants.

### Example 3: Confirmation of Reactivity of P32 Antigen to ASF Reference Serum

An antibody serum diagnostic kit prototype was manufactured using the P32 antigen protein prepared in Example 2.2, and reactivity and sensitivity tests were performed with serum provided by the Spanish ASF reference laboratory.

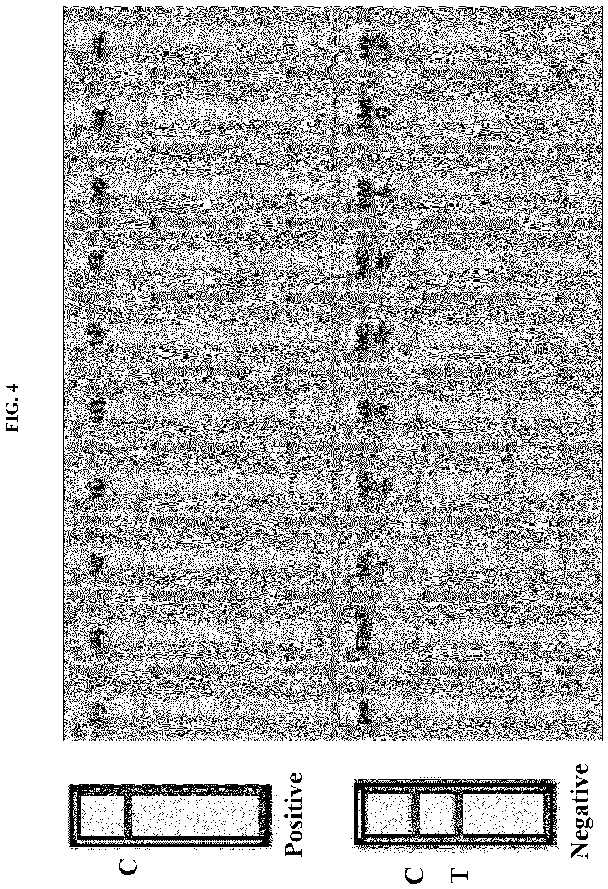

As a result, as shown in FIG. 4 and Table 1 below, the kit for ASF diagnosis of the present invention manufactured using the recombinant P32 antigen protein exhibited 100% identical positive and negative results matching the provided sample results, and it was also confirmed that sera set to the minimum positive limit (FIG. 4, limi; Table 1, Positive minimum limit Ref. serum) were determined as positive, showing excellent specificity and sensitivity.

**[Table 1]**

| No. | Serum (label in FIG. 4) | Infected by virus or not (actual) | Kit test results | Results match or not |
|---|---|---|---|---|
| 1 | Ref. serum 13 (13) | ○ | Positive | Match |
| 2 | Ref. serum 14 (14) | ○ | Positive | Match |
| 3 | Ref. serum 15 (15) | ○ | Positive | Match |
| 4 | Ref. serum 16 (16) | ○ | Positive | Match |
| 5 | Ref. serum 17 (17) | X | Negative | Match |
| 6 | Ref. serum 18 (18) | X | Negative | Match |
| 7 | Ref. serum 19 (19) | ○ | Positive | Match |
| 8 | Ref. serum 20 (20) | ○ | Positive | Match |
| 9 | Ref. serum 21 (21) | ○ | Positive | Match |
| 10 | Ref. serum 22 (22) | ○ | Positive | Match |
| 11 | Positive control Ref. serum (po) | ○ | Positive | Match |
| 12 | Positive minimum limit Ref. serum (limi) | ○ | Positive | Match |
| 13 | Negative serum 1 (Ne 1) | X | Negative | Match |
| 14 | Negative serum 2 (Ne 2) | X | Negative | Match |
| 15 | Negative serum 3 (Ne 3) | X | Negative | Match |
| 16 | Negative serum 4 (Ne 4) | X | Negative | Match |
| 17 | Negative serum 5 (Ne 5) | X | Negative | Match |
| 18 | Negative serum 6 (Ne 6) | X | Negative | Match |
| 19 | Negative serum 7 (Ne 7) | X | Negative | Match |
| 20 | Negative serum 8 (Ne 8) | X | Negative | Match |

The foregoing description of the present invention is provided for illustrative purposes only, and it will be understood by those of ordinary skill in the art to which the present invention pertains that the present invention may be easily modified into other particular forms without changing the technical spirit or essential characteristics of the present invention. Thus, the above-described embodiments should be construed as being provided for illustrative purposes only and not for purposes of limitation.

### INDUSTRIAL APPLICABILITY

A recombinant African swine fever virus antigen of the present invention not only can be efficiently produced using a plant, but can also be easily isolated and purified due to high water solubility thereof. In addition, since the antigen has high diagnostic sensitivity and specificity, a composition and kit for diagnosing African swine fever manufactured using the same can diagnose individuals infected by African swine fever early, and thus are expected to be highly industrially applicable.

## Claims

1. A recombinant vector for producing an antigen for the diagnosis of African swine fever, the recombinant vector comprising a polynucleotide encoding an African swine fever virus P32 protein consisting of an amino acid sequence of SEQ ID NO: 2, and being expressed in a plant.

2. The recombinant vector of claim 1, further comprising a polynucleotide encoding a chaperone binding protein (BiP) of SEQ ID NO: 3.

3. The recombinant vector of claim 2, wherein the polynucleotide encoding a BiP is located upstream of a 5' terminal of the polynucleotide encoding the P32 protein.

4. The recombinant vector of claim 1, further comprising a polynucleotide encoding polyhistidine of SEQ ID NO: 4.

5. The recombinant vector of claim 4, wherein the polynucleotide encoding polyhistidine is located downstream of a 3' terminal of the polynucleotide encoding the P32 protein.

6. The recombinant vector of claim 1, further comprising a polynucleotide encoding HDEL of SEQ ID NO: 6.

7. The recombinant vector of claim 6, wherein the polynucleotide encoding HDEL is located downstream of a 3' terminal of the polynucleotide encoding the P32 protein.

8. The recombinant vector of claim 1, further comprising:
a polynucleotide encoding a BiP of SEQ ID NO: 3;
a polynucleotide encoding polyhistidine of SEQ ID NO: 4; and
a polynucleotide encoding HDEL of SEQ ID NO: 6.

9. The recombinant vector of claim 8, wherein the recombinant vector has a polynucleotide encoding a BiP, a polynucleotide encoding the P32 protein, a polynucleotide encoding polyhistidine, and a polynucleotide encoding HDEL, which are sequentially linked to one another.

10. The recombinant vector of claim 8, wherein the recombinant vector comprises a nucleotide sequence of SEQ ID NO: 8.

11. A transgenic organism transformed with the recombinant vector of any one of claims 1 to 10.

12. The transgenic organism of claim 11, wherein the transgenic organism is a plant.

13. A recombinant African swine fever virus P32 protein produced using the recombinant vector of any one of claims 1 to 10.

14. The recombinant African swine fever virus P32 protein of claim 13, wherein the protein is water-soluble.

15. A composition for diagnosing African swine fever, the composition comprising, as an active ingredient, the recombinant African swine fever virus P32 protein of claim 13.

16. A kit for diagnosing African swine fever, the kit comprising, as an active ingredient, the recombinant African swine fever virus P32 protein of claim 13.

17. A method of diagnosing African swine fever, the method comprising detecting an antibody against an African swine fever virus through an antigen-antibody reaction in a biological sample derived from an animal other than a human by using, as an antigen, a recombinant African swine fever virus P32 protein produced using the recombinant vector of any one of claims 1 to 10.

18. A method of producing a recombinant antigen for the diagnosis of African swine fever, the method comprising:
(a) transforming a plant with the recombinant vector of any one of claims 1 to 10; and
(b) isolating and purifying a recombinant antigen from the plant.

19. A use of a recombinant African swine fever virus P32 protein produced using the recombinant vector of any one of claims 1 to 10 for diagnosing African swine fever.

20. A use of a recombinant African swine fever virus P32 protein produced using the recombinant vector of any one of claims 1 to 10 for preparing an agent used for the diagnosis of African swine fever.
